# EUROPEAN PATENT APPLICATION

(11) **EP 0 813 155 A1**
(43) Date of publication of application: **17.12.1997**
(21) Application number: 97200964.1
(22) Date of filing: 02.04.1997
(51) Int. Cl.: G06F 17/00

(54) **Disease management method and system**

(30) Priority: 10.06.1996 US 19011
(71) Applicant: SMITHKLINE BEECHAM CORPORATION, Philadelphia, PA 19101 (US)
(72) Inventor: Blissenbach, Henry F., Edina, Minnesota 55435 (US); Edgren, Bruce E., Edina, Minnesota 55435 (US); Chomilo, Frederick N., Edina, Minnesota 55435 (US); Eyer, Kathleen M., Edina, Minnesota 55435 (US)
(74) Representative: Connell, Anthony Christopher

(57) **Abstract**

A method for managing disease treatment in a population of patients undergoing drug therapy which comprises monitoring the patients' drug consumption through claims for pharmaceutical cost reimbursement and intervening in the disease treatment in accordance with predetermined intervention strategies, the particular intervention being dependent upon each patient's drug consumption.

## Description

### Field of the Invention

This invention is in the field of human healthcare and, more particularly, in the field of disease management.

### Background of the Invention

Asthma is one example of a disease that can be treated more effectively and more cost-effectively by designing a program to maximize physician prescribing of, and compliance with, current best medical practices (consistent with a given physician's professional judgment on a case-by-case basis.) Asthma is a serious health condition characterized by airway inflammation, airway irritability and bronchospasm. It affects persons of all ages but is most prevalent in geriatric adults and those under 18 years of age. The number of cases of asthma in developed countries has been rising at an alarming rate. Asthma is estimated to cost over $6 billion annually in the United States alone. Of this amount, approximately 45% is spent on inpatient hospital charges, 30% on medications, 14% on emergency room and outpatient hospital charges, and 13% for physician services. Recent research has demonstrated that the basic pathophysiology process of asthma is inflammation. As a result, asthma treatment has moved from episodic, symptomatic treatment to disease reduction and prevention.

The guidelines for asthma management in this country were defined by the National Asthma Education Program (NAEP) when it published "Guidelines for the Diagnosis and Management of Asthma" (Guidelines) in 1991. The Guidelines provide specific, comprehensive strategies for the diagnosis and management of pediatric and adult patients both in outpatient and inpatient settings. According to the Guidelines, asthma treatment should be individualized, include a comprehensive educational component, and focus on anti-inflammatory treatment to achieve the goals of asymptomatic nights, ability to exercise, and stable near normal peak expiratory flow rates. The Guidelines recommend a stepped approach to asthma therapy: 1) mild asthma should be treated with a beta agonist, 2) moderate asthma should be treated with the combination of beta agonist and an inhaled anti-inflammatory, 3) severe asthma should be treated with the combination of a beta agonist, inhaled anti-inflammatory, and daily oral corticosteroids. These guidelines are incorporated herein by reference as though fully set forth.

Healthcare costs in general are rising rapidly and the costs of treating asthmatics is no exception. In many cases, and in the case of asthma in particular, the costs of treating patients is not distributed evenly among the total population of patients, i.e., it costs more to treat some patients than others. This is due, at least in part, to the fact that some patients are not receiving appropriate therapies for their condition. This problem itself has several causes, including that some patients do not comply with prescribed treatment regimens, that some patients do not visit their doctors at appropriate times, and, in some cases, that some patients' doctors are not aware that a certain therapy regimen is likely to be more effective than their current regimen.

If patients are treated in accordance with therapy regimens proven to be effective for a given state of disease progression, then the total costs of treating the whole population of patients will decline. The theory is simple: if more patients are treated properly then the number of cases which progress to more serious stages of disease, which are more costly to treat, is reduced.

It is an object of this invention therefore to provide a method to maximize physician prescribing of, and compliance with, current best medical practices (consistent with a given physician's professional judgment on a case-by-case basis) by utilizing pharmaceutical cost reimbursement claims as an indicator of a patient's drug usage and condition and intervening in the disease treatment regimen of such patient on the basis thereof.

### Summary of the Invention

This invention provides a method for managing the treatment of patients having a given disease which comprises:
a) analyzing pharmaceutical cost reimbursement claims of a population of patients to identify the subpopulation of patients who are at risk and
b) intervening in the treatment of such subpopulation of at risk patients.

In a further embodiment, this invention comprises a method for managing disease treatment in a patient undergoing drug therapy which comprises:
a) monitoring the patient's ongoing drug utilization patterns through claims for pharmaceutical cost reimbursement to determine the efficacy of the patient's treatment regimen and
b) intervening in the disease treatment in accordance with a predetermined intervention strategy, the particular intervention being dependent upon the patient's drug utilization patterns.

This invention also comprises a computer-based system for managing disease treatment in a patient undergoing drug therapy for a given disease state which comprises:
a) a pharmaceutical claims database having discreet patient records showing pharmaceutical reimbursement claims,
b) means for analyzing the data for presence of intervention triggers and for identifying a corresponding intervention, and
c) means for effecting the intervention.

In yet another aspect this invention involves a computational device which can output a product useful to a physician or healthcare provider in managing the treatment of a disease in an identified patient population, wherein the device comprises at least one computer and one or more algorithms which:
a) collects pharmaceutical claims data and related patient data,
b) organizes said claims according to a predetermined disease state following a pre-programmed algorithm,
c) selects patient data for the predetermined disease state from said data using a pre-programmed algorithm,
d) outputs the selected patient data to a second database which contains pre-existing information on intervening in the treatment of the selected patients, and
   wherein said second database outputs physical or electronic information for intervening in the treatment of said patients, optionally pre-addressed to the patients' healthcare provider.

### Detailed Description of the Invention

The disease management method of this invention is intended to increase the number of patients within a given population of patients who receive, comply with and correctly administer appropriate therapies. The invention requires identifying preferred treatment regimens for given stages of disease progression. These may be published as medical guidelines or they may be developed by healthcare professionals for a given disease population.

In many cases, pharmaceutical utilization can be used as a surrogate marker for a given illness. For example, patients utilizing insulin can be presumed to be suffering from diabetes. At this simple level, such "drug utilization evaluation" can be used to identify a population of patients suffering a given disease and the patients or their physicians can be provided with educational materials regarding their disease and the treatment thereof.

At the next level, in accordance with this invention, within a given illness population, pharmaceutical utilization can further be used to identify an "at risk" subpopulation, i.e., a subpopulation of patients who appear likely to suffer exacerbations, e.g., because they are not responding in an ideal way to a given therapy or are not receiving a preferred treatment regimen considering the state of the art and the inferred condition of the patients. In these cases, a disease management program of the invention involves intervening in the treatment to improve patient compliance with a prescribed therapy or to modify the prescribed treatment regimen. Preferably, in the method of the invention, patients progress is regularly monitored over a period of time and interventions are made whenever required or appropriate. Intervention can be direct with the patient, or through the physician, or both, bearing in mind that each patient's physician is ultimately responsible for prescribing treatment regimens and for the care, health and safety of his or her patients.

Pharmaceutical utilization is inferred from analyses of pharmaceutical cost reimbursement claims. Based on the pharmaceutical use data, a treatment regimen statistically proven to be effective can be selected or compliance with proper administration methods can be improved. If a patient is already receiving the selected therapy, then intervention may not be required. If a patient is not receiving the selected therapy, then the patient will be treated in accordance with the selected therapy, e.g., by modifying the therapy or by improving the patients compliance with administration protocols.

The "triggers," i.e., the pharmaceutical utilization events, or patterns, from which one infers that a given subset of patients is at risk and for whom intervention is appropriate are determined on a disease-by-disease basis and are updated as new therapies become available and as new data are developed and evaluated. In a preferred embodiment of the invention, pharmaceutical use data are analyzed and compared with patients' health records to identify new triggers for intervention.

The method of the invention is most effective with a relatively large patient population. Thus, in practicing the method of the invention, the number of patients in the program is preferably at least 500, more preferably at least 1,000, and most preferably at least 2,000. In such large patient populations, use of the method of the invention can result in a 25% or greater increase in the number of patients receiving (and complying with) preferred therapies. Preferably, the increase will be 35% or greater.

The method of the invention typically involves, in addition to the patients, a data provider, a case manager, and multiple physicians. The data provider provides the pharmaceutical use data. As described above, the pharmaceutical use data can be derived from a pharmaceutical reimbursement claims data such as are compiled by prescription insurance providers.

The case manager does not diagnose disease or prescribe treatment regimens. Medical diagnosis and treatment is the sole responsibility of licensed physicians. In the method of this invention, the case manager facilitates patient treatment by identifying to physicians patients who are likely to benefit from a change in therapy and by suggesting therapies to the physicians, and by providing educational and treatment compliance assistance to patients (with the concurrence of the treating physicians).

The case manager identifies patients receiving pharmaceutical therapy and, more importantly, the subset of these who are not being treated in accordance with the preferred treatment regimen for the patient's disease state. This population of patients is very relevant to this invention; from this population, the treatment regimen of those patients who are not receiving the preferred therapy regimen can be changed to the preferred treatment regimen.

In some embodiments of this invention, the roles of the case manager are divided among multiple persons or entities. For example, one entity can identify patients at risk for becoming "high-cost" patients, another entity can contact physicians with this information and with treatment advice as well as with patient educational materials and treatment compliance devices, and yet a third entity can contact physicians directly. The important point is that as a result of carrying out the method of the invention a larger number of patients receive appropriate therapies than would otherwise and that as a consequence a smaller number of patients suffer from serious disease progression requiring extraordinary, and expensive, care.

The method of the invention typically involves at least 100 treating physicians, but is also effective with larger numbers of physicians, e.g., 250 to 500 physicians, or more.

In a preferred embodiment of the invention, the above-described disease management program is supplemented by a program based on hospitalization and emergency room visits. This supplemental program comprises inferring disease progression and efficacy of treatment based on hospitalization and emergency room visits as further described herein below.

The method of the invention is typically carried out with the assistance of an electronic database for storage, and retrieval, of at least the following data from pharmaceutical reimbursement claims:
a) patient identifier
b) drug prescribed
c) drug dosage
d) amount of drug
e) duration of drug therapy
f) dates of recent prescription fills/refills
g) provider identifier.
The data are stored preferably in machine-readable form and are recoverable in discreet, searchable fields with a discreet record for each patient. Each record also preferably comprises a field for noting whether or not one or more case management interventions as described herein have been undertaken. The data are stored in a computer and accessed through customized database utilization software. Such software will provide searching and reporting (display, printing, and electronic distribution) capability.

To make database use efficient, the database utilization software should have the capability of eliminating redundant entries, for eliminating entries for patients who have become ineligible and for silencing records for which a case management intervention has been undertaken within a preset period of time. A preferred embodiment of this invention comprises constructing such database and utilizing such database access software in a computer to prompt or directly effect disease management intervention as described herein, e.g., to prompt a case manager to advise a physician (or to directly issue such advice) as described herein. Form letters, in hard copy or in e-mail form, for patient education and for advising physicians can be prepared for use in the method of the invention. Or this information can be provided through a distributed system, e.g., an Internet home page.

The particular intervention can also be triggered directly by the computer system by programming the computer to analyze the pharmaceutical claims data for presence of intervention triggers, i.e., claims patterns that correspond to a given intervention in accordance with a predetermined intervention strategy.

Alternatively, a single computer, or two or more, can be programmed so that it collects or can be fed pharmaceutical claims data, cleans up the data and sorts it according to a preset algorithm, identifies a patient population on demand, outputs that data to a second system running on the same computer or on a second computer which is programmed to recognize the patient data being input, is capable of selecting information useful for intervening in that patient population and collating the patient information with the intervention information and outputting the combined product for use by the healthcare provider or another.

Any computer can be used in the practice of this invention. Obviously the practitioner will wish to select a device which is capable of handling the several operations this invention needs to have carried out. Processing large amounts of data rapidly is desirable. Memory should be adequate for handling data input and storage. For example if data is obtained from scanned images, substantially more disk storage space will be needed as compared with processing an ASCII file. The selected hardware should be capable of accepting data by any means, e.g., telecommunications lines, tape or disk drive, scanned images, or manual input. The hardware should also be capable of any type of output, printing, facsimile, or electronic communications such as e-mail or Internet interface.

It is expected that this invention could be practiced on a state of the art PC or Macintosh. The choice of PC or Mac is left to the preference of the practitioner. A PC with Pentium chip operating 120 Mhz or higher, at 8 megabytes of RAM, and a 1 gigabyte hard disk should provide useful computational capability. Increased computing power can be had by going to a workstation such as those sold by Sun Microsystems or Hewlett Packard, for example. Mainframe systems, particularly IBM minis or mainframes or Digital Equipment Corp. VAX system could be used as well. No preference is expressed here.

Any operating system can be used. Graphical user interface-based systems are now preferred. Microsoft Windows 6.0, Windows 95 or Windows NT should be useful. If the same device will be used for collecting and sorting data as is used for the output step, an operating system capable of multi-tasking, such as UNIX, may be useful.

As for database software for collecting data, or for marrying patient data outputs with intervention information, it can be selected from any off the shelf relational data base package. For PCs any of the well known relational data bases such as Access by Microsoft can be used. This applies to handling the intervention information as well. A text and/or graphical package may be included in the system as well to handle the intervention information. Microsoft Word, or WordPerfect are examples of text software.

Such database and utilization software are useful for a variety of disease management programs based on pharmaceutical reimbursement claims data. Examples of disease or other abnormalities for which disease management programs of the invention are suitable include: asthma, heart disease, osteoporosis, diabetes, ulcers and depression.

One exemplary embodiment of this invention comprises a method for managing the treatment of a population of asthmatic patients which comprises:
(1) identifying and selecting the subset of patients being treated with high dose inhaled beta agonist therapy
(2) categorizing the identified patients into the following categories:
   Category 1: patients treated with standard inhaled antiinflammatory drug therapy and treated with standard pulsed oral steroid therapy
   Category 2: patients treated with standard inhaled antiinflammatory drug therapy and not treated with standard pulsed oral steroid therapy
   Category 3: patients not treated with standard inhaled antiinflammatory drug therapy and treated with standard pulsed oral steroid therapy
   Category 4: patients not treated with standard inhaled antiinflammatory drug therapy and not treated with standard pulsed oral steroid therapy
(3) intervening in the therapy of the patients based on category as follows:
   Category 1: evaluate the medical condition of each patient and, if appropriate in accordance with standard medical practice, treat the patient with an alternative or supplemental therapy
   Category 2: treat the patients with a therapy which preferably includes standard pulsed oral steroid therapy supplemental to the antiinflammatory therapy
   Category 3: treat the patients with standard inhaled antiinflammatory drug therapy supplemental to the steroid therapy
   Category 4: treat the patients with standard inhaled antiinflammatory drug therapy and with a therapy which preferably includes standard pulsed oral steroid therapy.

Note that by "not treated" is meant that the therapy has not been prescribed or has been prescribed but in insufficient amounts, or has been prescribed in sufficient amounts but the patient is not complying with the prescribed treatment regimen.

The case manager identifies those asthma patients that are being treated on average with 8 puffs or more of an inhaled beta agonist per day over a 90 day time period. It will be appreciated that, in practice, treatment and intervention are at the discretion of qualified healthcare practitioners. Therefore, not all patients in the 4 identified categories will receive the recited treatment interventions. In particular, for example, a Category 1 patient will normally be examined by a qualified healthcare practitioner and will receive a supplemental or alternative therapy only if warranted in the opinion of the examining healthcare practitioner. By way of further example, a Category 2 patient may not be treated with the steroid therapy if, *e*.*g*., in the opinion of the patient's physician there has been no exacerbation in the disease state which is unrelated to improper administration of the antiinflammatory therapy or which otherwise does not warrant treatment intervention. Or, a qualified healthcare practitioner may prescribe different alternative, or further supplemental, therapies such as, *e*.*g*., sustained oral steroids, oral theophylline, environment changes, *etc*. The principal goal of this invention is to assure that within a population of asthmatics those patients who are likely to benefit from treatment intervention are identified and examined by qualified healthcare practitioners who have knowledge of current best practices.

The preferred method of treatment for patients being treated on average with 8 puffs per day or more of an inhaled beta agonist over a 90 day time period comprises treatment with an inhaled anti-inflammatory and with additional therapy which typically includes pulsed oral steroid therapy, in addition to the inhaled beta-agonist therapy. The goals for the preferred method of treatment are to reduce each patient's requirement for inhaled beta agonist therapy and to reduce each patient's utilization of pulsed oral steroids.

The Guidelines define the preferred therapy for the treatment of asthma. Patients who are being treated in accordance with the preferred therapy should nonetheless be periodically examined. In some cases, such patients do not respond favorably to the preferred therapies. In such cases, patients should receive alternative therapies. For example, an alternative therapy might comprise other drug therapy, in-home nursing care or hospitalization. Other drug therapy can include the use of oral methylxanthines (e.g., theophylline products), long-acting beta agonists and chronic oral steroids. In-home nursing care can provide on-site education with regard to disease, triggers and drug therapy and encourage and monitor compliance to preferred treatment regimens. Hospitalization is always available and is recommended for acute exacerbations, treatments and procedures.

Pharmacy claims (drug patterns in addition to those aforementioned), demographic data and seasonal variation may also be used to identify individuals with asthma who are at risk for extraordinary utilization of healthcare resources. These populations may include children under 10 years of age and pregnant women.

The following example is illustrative of the invention and is not intended to limit the scope of the invention which includes all embodiments of the inventions as hereinabove described.

Pharmacy claims data are analyzed by a case manager to identify individuals receiving medications indicative of asthma whose regimens appear inconsistent with national asthma guidelines. The Guidelines recommend that individuals requiring routine use of high dose beta-agonist inhalers should receive a prescription for an inhaled anti-inflammatory medication. The pharmacy claims analysis identifies all active members who have a three month prescription claim history indicating high dose, i.e., ≥ 8 puffs/day, beta-agonist metered dose inhaler use of albuterol (Ventolin, Proventil), metaproterenol (Alupent, Metaprel), bitolterol (Tornalate), isoetharine (Bronkometer), isoproterenol (Medihaler-Iso, Mistometer), isoproterenol/phenylephrine (Duo-Medihaler), salmeterol (Serevent), terbutaline (Brethaire), or pirbuterol (Maxair). The severity of illness (Classifier 1 - 6) in high dose beta-agonist users is then stratified based on the utilization of an inhaled anti-inflammatory medication (Intal, Tilade, Beclovent, Vanceril, Decadron Respihaler, Azmacort, Aerobid or Aerobid-M) and oral corticosteroids (cortisone, hydrocortisone, prednisone, triamcinolone, prednisolone, methylprednisolone, or dexamethasone) during the same time period. The use of oral corticosteroids implies an increased severity in their condition. The specific asthma medications are identified based on NDC codes, generic codes and Therapeutic Class codes.
Classifier I individuals are active members who have used ≥ 8 puffs/day of beta-agonist therapy with ≥ 4 puffs/day of inhaled anti-inflammatory medications, and utilized oral corticosteroids during the previous three months.
Classifier II individuals are active members who have used ≥ 8 puffs/day of beta-agonist therapy with ≥ 4 puffs/day of inhaled anti-inflammatory medications without oral corticosteroids during the previous three months.
Classifier III individuals are active members who have used ≥ 8 puffs/day of beta-agonist therapy with < 4 puffs/day of inhaled anti-inflammatory medications, and utilized oral corticosteroids during the previous three months.
Classifier IV individuals are active members who have used ≥ 8 puffs/day of beta-agonist therapy with < 4 puffs/day of inhaled anti-inflammatory medications without oral corticosteroids during the previous three months.
Classifier V individuals are active members who have used ≥ 8 puffs/day of beta-agonist therapy without inhaled anti-inflammatory medications, and utilized oral corticosteroids during the previous three months.
Classifier VI individuals are active members who have used ≥ 8 puffs/day of beta-agonist therapy without inhaled anti-inflammatory medications nor oral corticosteroids during the previous three months.

The patients' physicians are provided with an asthma prescription medication profile and inferred health status for each identified individual in their care with a description of potential inconsistencies between the patient's prescription history and guideline recommendations. Not only does this process help identify patients possibly suitable for therapy modification, but it provides valuable information to those physicians who may be prescribing in compliance with guidelines, but whose patients fail to take the medications in the manner prescribed. A response form may be completed by the physician and mailed or faxed to the case manager describing the physician's decisions (changes in therapy, education, etc.). The physician also has the opportunity to request patient reinforcement of the care plan through educational support, e.g., through direct telephone contact and patient mailings of educational materials. After allowing a three month interval for the physician to take action with the patient and/or refer them into a patient education program for further education, the pharmacy claims analyses are repeated to assess therapy changes. The case manager should have the capability of generating guideline compliance profiles for physicians which can be used to provide valuable feedback on physician performance and asses the impact of the behavior modification programs.

More specifically, the case manager advises the healthcare provider, i.e., the physicians treating the patients, that their patients are or are not, for whatever reason, receiving the preferred asthma therapies. Based on this advice, physicians are expected to effect a change in their patients' therapies either by prescribing a different therapeutic regimen or by attempting to effect changes in their patients' behaviors, as follows:
Category 1 (corresponds to Classifier I): evaluate the medical condition of each patient and, if appropriate in accordance with standard medical practice, treat the patient with an alternative therapy
Category 2 (corresponds to Classifier II): treat the patients with therapy which includes standard pulsed oral steroid therapy
Category 3 (corresponds to Classifiers III and V): treat the patients with standard inhaled antiinflammatory drug therapy
Category 4 (corresponds to Classifiers IV and VI): treat the patients with standard inhaled antiinflammatory drug therapy and with therapy which includes standard pulsed oral steroid therapy.

Participating patients may also receive case management from the case manager. Case management strategies focus on enhancing the individual's or caregiver's knowledge of the disease, triggers for acute exacerbations of asthma, and therapy (medications and inhaler techniques). A case manager may directly contact the patient or caregiver by telephone at a minimum of once a month for six months to assess the patient's knowledge base and self-management skills and provide additional learning opportunities. Education is tailored for the special needs of adults, adolescents and caregivers of children less than 12 years old with asthma. Tools include patient educational letters, brochures, video and audio tapes, peak flow meters, patient diaries to monitor performance, as well as personal training and access to support groups. These tools are utilized for the different patient populations at various stages to enhance the results of the program strategies.

A disease management program based on medical claims can be used to supplement the above-described disease management program based on pharmacy claims. Because the pharmacy and medical claims identification and strategies differ, the patient will be offered all the corresponding components for which they qualify. In addition, patients participating in one component of the program may be referred into the other by the physician, program educators, or case managers. The program avoids duplication of effort through a comprehensive tracking system which involves individualized records. Such supplemental program comprises:
(1) identifying and selecting the subset of patients who have visited hospitals or hospital emergency rooms for treatment of asthma
(2) categorizing the identified patients into the following categories:
   Level 1: patients who have been diagnosed with asthma during a 12 month period but who have not had an asthma-related hospitalization nor an emergency room visit during this period
   Level 2: patients who have been diagnosed with asthma during a 12 month period and who have had an asthma-related emergency room visit during this period
   Level 3: patients who have been diagnosed with asthma during a 12 month period and who have had an asthma-related hospitalization during this period
(3) providing disease management counseling to these patients based on category as follows:
   Level 1 (mild) asthmatics receive patient literature during the first year of the program. Educational brochures provide an opportunity for increased understanding of the disease state, present the possible effects of the progression of the condition and encourage compliance with the treatment regimen prescribed by their physician. Reassessment of medical claims data is done periodically to identify patients who move from this category to different severity levels. Also included in the mailing is a business reply card survey form which permits individuals to request additional asthma related information. One may opt to distribute the materials at health fairs or through mailings to all individuals. This generalized approach involves a higher cost with less return.
   Level 2 (moderate) asthmatics also receive the patient literature described above. In addition, patients receive, through their physicians, educational kits designed to empower the patient to manage their disease consistently with instructions from their physicians. These kits include a peak-flow meter, diary and a video tape to encourage proper use of their inhalers. The physician will be responsible for distributing the kits to their patient population. (The program does not guarantee that the appropriate individuals, nor even client's members, will receive the kits). *Pediatric* level II asthmatics will also receive invitations to asthma education workshops sponsored as described for Level 3 patients below.
   Level 3 (severe) asthmatics (and pediatric level IIs) receive invitations to three educational workshops during the first year of the program. The educational program is an National Heart, Lung & Blood Institute based comprehensive training and patient education program designed for the office nurse and/or patient educator. The educators will have an understanding of asthma therapies and treatment protocols, and be trained to effectively educate asthma patients to participate in promoting positive outcomes. Participants attending the educational workshops will receive either a Pediatric or Adult "Counseling Bag" which contains a visual aid identifying the symptoms of asthma, a personalized patient asthma management plan, a pre- and post-counseling assessment of asthma knowledge, an asthma diary and a patient resource guide.

## Claims

1. A method for managing the treatment of patients having a given disease which comprises
(a) analyzing pharmaceutical cost reimbursement claims of a population of patients to identify the subpopulation of patients who are at risk and
(b) intervening in the treatment of such subpopulation of at risk patients.

2. The method of claim 1 wherein the pharmaceutical cost reimbursement claims are monitored by analyzing claims data from a pharmaceutical claims database.

3. The method of claim 2 which comprises a program for managing disease treatment in a patient population of at least 500 patients.

4. The method of claim 3 wherein the database comprises discreet patient records and each record has at least the following searchable fields:
a) patient identifier
b) drug prescribed
c) drug dosage
d) amount of drug
e) duration of therapy
f) dates of recent prescription fills/refills
g) provider identifier.

5. The method of claim 4 wherein the database is operatively linked to a computer wherein the computer is preprogrammed to analyze the data and to effect a case management intervention strategy.

6. The method of claim 5 wherein the database is programmed to eliminate entries for patients who have become ineligible and to silence records for which a case management intervention has been undertaken within a preset period of time.

7. The method of claim 6 wherein the case management intervention is effected through a case manager.

8. The method of claim 6 wherein the case management intervention is effected directly by the preprogrammed computer.

9. The method of claim 7 wherein the case management intervention comprises prompting a case manager to advise a physician or optionally directly issuing such advice to a patient.

10. The method of claim 8 wherein the case management intervention comprises prompting a case manager to advise a physician (or directly issuing such advice) to a patient.

11. The method of claim 9 wherein the advice is issued to a patient to comply with the prescribed treatment regimen or to submit to examination by a physician.

12. The method of claim 10 wherein the advice is issued to a patient to comply with the prescribed treatment regimen or to submit to examination by a physician.

13. The method of claim 11 wherein the advice is issued to the healthcare provider and is to modify the prescribed treatment regimen or to submit the patient to a physician's examination.

14. The method of claim 12 wherein the advice is issued to the healthcare provider and is to modify the prescribed treatment regimen or to submit the patient to a physician's examination.

15. The method of claim 1 which further comprises monitoring medical costs reimbursement data for the patient and wherein the medical claims data together with the pharmaceutical claims data are jointly used to determine the disease treatment intervention.

16. A method for managing disease treatment in a patient undergoing drug therapy which comprises:
(a) monitoring the patient's ongoing drug utilization patterns through claims for pharmaceutical cost reimbursement to determine the efficacy of the patient's treatment regimen and
(b) intervening in the disease treatment in accordance with a predetermined intervention strategy, the particular intervention being dependent upon the patient's drug utilization.

17. A computer-based system for managing disease treatment in a patient undergoing drug therapy for a given disease state which comprises:
a) a pharmaceutical claims database having discreet patient records showing pharmaceutical reimbursement claims
b) means for analyzing the data for presence of intervention triggers and for identifying a corresponding intervention
c) means for effecting the intervention.

18. A computational device which can output a product useful to a physician or healthcare provider in managing the treatment of a disease in an identified patient population, wherein the device comprises at least one computer and one or more algorithms which:
a) collects pharmaceutical claims data and related patient data,
b) organizes said claims according to a predetermined disease state following a pre-programmed algorithm,
c) selects patient data for the predetermined disease state from said data using a pre-programmed algorithm,
d) outputs the selected patient data to a second database which contains pre-existing information on intervening in the treatment of the selected patients, and
wherein said second database outputs physical or electronic information for intervening in the treatment of said patients, optionally pre-addressed to the patients' healthcare provider.
